# EUROPEAN PATENT SPECIFICATION

(11) **EP 1 552 848 B1**
(45) Date of publication and mention of the grant of the patent: **22.10.2008**
(21) Application number: 03736272.0
(22) Date of filing: 26.06.2003
(51) Int. Cl.: A61K 39/395, A61K 35/74, A61P 33/02, A23K 1/18, A23K 1/16

(54) **COMPOSITIONS AGAINST CHICKEN COCCIDIOSIS**
ZUSAMMENSETZUNGEN GEGEN COCCIDIOSIS BEI HÜHNERN
COMPOSITIONS DIRIGEES CONTRE LA COCCIDIOSE DU POULET

(30) Priority: 28.06.2002 JP 2002189137
(43) Date of publication of application: 13.07.2005
(73) Proprietor: Ghen Corporation, Gifu-shi Gifu-ken 501-1132 (JP)
(72) Inventor: KODAMA, Yoshikatsu, Gifu-shi, Gifu 501-1101 (JP); YOKOYAMA, Hideaki, Gifu-shi, Gifu 501-1101 (JP); NGUYEN, Sa Van, Gifu-shi, Gifu 501-1101 (JP)
(74) Representative: Ziebig, Marlene
(86) International application number: PCT/JP2003/008132
(87) International publication number: WO 2004/002527

(56) References cited:
- EP-A1- 0 344 808
- EP-A2- 0 135 073
- EP-A2- 0 164 176
- EP-A2- 0 439 056
- WO-A-94/21284
- WO-A-98/08540
- JP-A- 7 255 386
- JP-A- 8 040 935
- JP-A- 10 182 483
- JP-A- 62 215 534
- JP-B2- 2 615 673
- SMITH N C ET AL: "Maternal transfer of antibodies induced by infection with Eimeria maxima partially protects chickens against challenge with Eimeria tenella" PARASITOLOGY, vol. 109, no. 5, 1994, pages 551-557, XP008056448
- LILLEHOJ H S ET AL: "A RECOMBINANT EIMERIA PROTEIN INDUCING INTERFERON-GAMMA PRODUCTION: COMPARISON OF DIFFERENT GENE EXPRESSION SYSTEMS AND IMMUNIZATION STRATEGIES FOR VACCINATION AGAINST COCCIDIOSIS" INTERNATIONAL WORKSHOP ON PHYSICS AND ENGINEERING IN MEDICAL IMAGING, vol. 44, no. 2, April 2000 (2000-04), pages 379-389, XP009041774
- BABA E ET AL: "Clostridial population and the intestinal lesions in chickens infected with Clostridium perfringens and Eimeria necatrix" VETERINARY MICROBIOLOGY, vol. 54, no. 3-4, 1997, pages 301-308, XP002356605 ISSN: 0378-1135
- PATENT ABSTRACTS OF JAPAN vol. 015, no. 377 (C-0870), 24 September 1991 (1991-09-24) & JP 03 151853 A (KANEBO LTD; others: 01), 28 June 1991 (1991-06-28)
- LILLEHOJ H S ET AL: "AVIAN COCCIDIOSIS. A REVIEW OF ACQUIRED INTESTINAL IMMUNITY AND VACCINATION STRATEGIES" AVIAN DISEASES, AMERICAN ASSOCIATION OF AVIAN PATHOLOGISTS, KENNET SQ., PA, US, vol. 44, no. 2, April 2000 (2000-04), pages 408-425, XP008044581 ISSN: 0005-2086

## Description

### Technical Field

This invention relates to a composition which is effective in preventing and treating chicken coccidiosis. To be more specific, this invention relates to a composition which has excellent prophylactic and therapeutic effects for coccidiosis of chickens, which is effective in preventing the infection of chicken coccidiosis, which is effective in improving clinical conditions such as body weight loss associated with the chicken coccidiosis, or which is highly effective in treating the chicken coccidiosis.

### Background Art

Chicken coccidiosis (coccidiosis in chickens) is caused by infection with a protozoan of genus Eimeria. In the case of chickens, symptoms and habitat of the parasite differ depending on the species of the Eimeria. The Eimeria species that are pathogenically important are the following 5 species, namely, Eimeria tenella, E. necatrix, E. maxima, E. brunetti, and E. acervulina, and other known Eimeria species include E. mitis, E. praecox, E. hagani, and E. mivati. In the case of quails, the coccidiosis is known to be caused by E. uzura, E. tsunodai, and the like; in the case of turkey, E. meleagrimitis, E. adenoides, and E. gallopovonis are known to be responsible; and in the case of guinea fowl, E. grenieri and E. numidae are known to be responsible. The coccidiosis is one of the chicken diseases that make most devastating damage, and the infection is established by oral intake of the oocyst. The symptoms include various types of diarrhea, anorexia, asitia, and weight loss, and the infected bird often dies. The birds are infected irrespective of their age (by day), sex, or variety. Although the coccidiosis is more frequently found in free range chicken coop, outbreak of coccidiosis has been recently reported also from egg-laying hens of multi-layered cages. It is also to be noted that the symptoms of coccidiosis are more serious when the bird is simultaneously infected with Clostridium perfringens. Prevention of coccidiosis is generally accomplished by preventing cage contamination by oocysts, and by the use of anticoccidial drugs. Various synthetic anticoccidial drugs for birds and ionophorous antibiotics are used as preventive anticoccidial drugs, and a sulfa drug or a mixture of a sulfa drug and a pyrimidine drug are used for the treatment. Vaccines are also used on commercial bases.

In the case of chickens, the anticoccidial drugs can be used only within 10 weeks after hatchling, and in the case of a broiler chickens, it is mandatory to stop the drug administration at least 7 days before the shipping because eggs and meats are allowed to be shipped only after certain period has passed after the last administration of the anticoccidial drugs. It is this period during which use of the anticoccidial drugs are prohibited that outbreak of chicken coccidiosis is likely to develop. Another problem is increase of drug resistant chicken coccidium, and each farm, therefore, needs to select anticoccidial drugs that are effective for their farm. There was a time when a quite large number of anticoccidial drugs for chicken coccidiosis were developed. However, use of the anticoccidial drugs have been recently restricted in consideration of the action of the residual anticoccidial drugs, adverse effects on human and other animals, emergence of drug resistant chicken coccidium, and the like. In the case of Europe, use of anticoccidial drugs will be banned by the year 2007. Use of anticoccidial drugs has also been strictly prohibited in Japan because of the enforcement of the regulatory law on feed additives.

Concerning live vaccines for oral administration, attempts have been made to systematically immunize chickens with the strain of weak toxicity, thereby preventing infection by a highly toxic strain. An example of such live vaccine is the one comprising a mixture of oocysts from several species, which is administered to chicks so that the chicks will be infected to a mild non-fatal degree and acquire immunity. However, there is a nonnegligible downside in this type of avian coccidium live vaccine that the chicken farm is left contaminated. On the other hand, inactivated vaccines produce only insufficient amount of antibody for preventing the infection, and are not sufficiently effective. It is also to be noted that, the vaccine needs a period of several weeks before production of the protective antibody even if chicks were immunized, and this period is far too long for use in broiler chicks.

When a chicken is suffering from chicken coccidiosis, enteric flora of the chicken becomes disturbed and symptoms like diarrhea are induced. This diarrhea continues for a particularly long period to cause physical exhaustion as well as deterioration of immunity. The birds are then subject to suffer from necrotizing enterocolitis.

Japanese Patent Nos. 2548115, 2698778, and 2698779 disclose passive immunization of mammals using an avian antibody. This passive immunization is used in the course when a xenoantibody (from chicken egg) is administered for the purpose of passive immunization (and in particular, by injection or other parenteral administration), and in this process, the animals are preliminarily given a food containing an antibody so that oral immunotolerance is established before the prophylactic or therapeutic administration of an antibody, and thereby preventing anaphylactic shock and serum sickness. Accordingly, this process is absolutely different from the idea of the present invention wherein protection of the animal is sought by oral passive immunization. The inventors of the present invention also filed a patent application (Japanese Patent No. 2034005) directed to an oral prophylactic or therapeutic agent for colibacillosis of preweaning pig or cow, which is produced by immunizing a chicken with at least one antigen selected from 987P, K88, and K99 antigens of enterotoxigenic E. coli which is responsible for porcine colibacillosis and K99 antigen of enterotoxigenic E. coli which is responsible for bovine colibacillosis, and recovering the antibody from the egg produced by the immunized chicken; and this agent contains a polyclonal antibody specific for such antigen. The inventors of the present invention have also filed a patent application (Japanese Patent No. 2615673) directed to a material for suppressing a food poisoning bacterium in edible fowl, which comprises whole egg, egg yolk, or antibody-containing fraction of the egg produced by a chicken which has established an immunity by preliminarily inoculation of the food poisoning bacterium, and which contains an antibody specific for the food poisoning bacterium. While these patents may disclose usefulness of the chicken egg antibody, they only disclose protection of an animal against a bacterium by oral passive immunization, and not the protection against a protozoan. To this day, the idea of using passive immunization for protozoan remains unknown, while the idea of using the chicken egg antibody for orally inducing passive immunization against bacteria or virus may have been known to the art.

It is also to be noted only few articles report passive immunization of birds against chicken coccidium while many articles disclose vaccines using various antigens against chicken coccidium. There is an article wherein a murine monoclonal antibody against gametocytes of Eimeria maxima is produced to confirm passive immunization against chicken coccidiosis (Infection and Immunity, 56: 972-976, 1988, Infection and Immunity, 58: 557-562, 1990). In this article, however, the test is conducted by intravenous injection of the murine monoclonal antibody, and not by oral administration, and accordingly, this article is far from being practical.

Smith et al. (Database Biosis, Philadelphia, PA, USA, 1994, PREV199598078719) disclose a maternal transfer of IgG antibodies to hatchlings via egg yolk and confer a high degree of maternal immunity against homologous challenge of Eimeria maxima and partial immunity against Eimeria tenella. D1 does not describe an oral administration of antibodies to hatchlings.

Lillehoj et al. (Int. Workshop Phys. Eng. Med. Imaging, vol. 44, no. 2, Apr 2000, 379, XP009041774) disclose a monoclonal antibody used in the cross-species immuno-staining of sporozoites and merozoites of E. acervulina, E. tenella and E. maxima. The antigen used to raise said monoclonal antibody was shown to elicit an active anti-E. acervulina immune response.

WO 94/21284 discloses a beneficial effect of a combination of a pro-biotic lactobacilli with an antibody pretreated with a proteolytic enzyme in the treatment of E. coli-mediated diarrhoeal intestinal diseases.

WO 98/08540 discloses neutralizing polyclonal antibodies that are directed against proteins from the bacterium Clostridium botulinum and derived from eggs of hens. The antibodies are used for the treatment of hamsters, but not for birds.

Baba et al. (Database Biosis, Philadelphia, PA, USA, 1997, PREV199799519645) teach that E. necatrix and C. perfringens act synergistically in creating pathology.

JP 03 151853 discloses a composition containing antibodies directed against Clostridium perfringens, which are produced in egg yolk. The antibodies may be used as food additives to ameliorate C. perfringens related intestinal disorders.

### Disclosure of Invention

In view of the situation as described above, the inventors of the present invention has made an intensive study, and found that, when a chicken, is immunized with an antigenic outermembrane protein as an antigen which has an immunogenicity commonly shared among the sporozoit and the merozoite of Eimeria acervulina, with coccidiosis in chickens in order to produce an anti-chicken coccidiosis antibody, then the antibody in the egg produced by such bird is administered to a chicken, the antibody attaches to the sporozoit or the merozoite of the protozoan of the Eimeria species to inhibit attachment and invasion of the sporozoit or the merozoite to epithelial cell, thereby destroying pathogenicity of the sporozoit and the merozoite. The inventors of the present invention have also found that, when the anti-chicken coccidiosis antibody of the present invention is administered in combination with a lactic acid bacterium, excellent prophylactic and therapeutic effects for chicken coccidiosis is achieved through improvement of enteric flora and enhancement of immunity, and through more efficient action of the antibody of the present invention. The inventors also found that, when the anti-chicken coccidiosis antibody of the present invention is administered in combination with an antibody obtained from an egg of a chicken immunized with Clostridium perfringens, excellent prophylactic and therapeutic effects for chicken coccidiosis is attained through prevention of the worsening of the symptoms according to mixed infection by the Clostridium perfringens, and through more efficient action of the antibody of the present invention. The present invention has been completed on such findings.

This invention includes the inventions as described below.
(1) An anti-chicken coccidiosis composition for oral administration comprising an antibody obtained from an egg of a chicken immunized with an antigenic outermembrane protein having a common immunogenicity shared among sporozoit and merozoite of Eimeria acervulina, which is associated with chicken coccidiosis.
(2) A composition according to the above (1) further comprising a lactic acid bacterium.
(3) A composition according to the above (1) or (2) further comprising an antibody obtained from an egg of a chicken immunized with Clostridium perfringens.
(4) A composition according to any one of the above (1) to (3) which is used for prevention or treatment of chicken coccidiosis.
(5) An avian feed comprising the composition of any one of the above (1) to (4).

The present invention is described in further detail.

The antigen used in the present invention is an antigenic outermembrane protein which has an immunogenicity commonly shared among sporozoit and merozoite of Eimeria acervulina, which is associated with chicken coccidiosis.

Since this antigen has an immunogenicity commonly found in sporozoit and merozoite of Eimeria acervulina, the product produced by using this antigen is effective.

Examples of such antigens which may be used include the soluble protein (F3) or 3-1E protein with the size of 18 to 27 kilodaltons described in Avian Diseases, 44: 379-389, 2000; the merozoite protein with the size of 21 kilodaltons described in J. Parasitol., 84: 654-656, 1998; and the protective fraction protein (FV) described in Infection and Immunity, 59: 1271-1277, 1991.

The antigenic outermembrane protein can also be synthesized by solution phase peptide synthesis, solid phase peptide synthesis, or other method employed in the peptide synthesis, and if desired, the synthesis may be accomplished by using an automated peptide synthesizer. The synthesis may be carried out in accordance with the procedures described in "Lectures on Biochemical Experiments, 1, Chemistry of Proteins IV" edited by The Japanese Biochemical Society, published from Tokyo Kagaku Dojin, 1975; Izumiya et al, "Fundamentals and Experiments of Peptide Synthesis", Maruzen, 1985; "Lectures on Biochemical Experiments, Second Series, 2, Chemistry of Proteins, Second Volume" edited by The Japanese Biochemical Society, published from Tokyo Kagaku Dojin, 1987; and the like.

The antigenic outermembrane protein may also be prepared from the DNA or the RNA having corresponding nucleotide sequence by using a genetic engineering technique. (For example, see "Lectures on Biochemical Experiments, Second Series, 1, Methods in Gene Study I" edited by The Japanese Biochemical Society, published from Tokyo Kagaku Dojin, 1986; "Lectures on Biochemical Experiments, Second Series, 1, Methods in Gene Study II" edited by The Japanese Biochemical Society, published from Tokyo Kagaku Dojin, 1986; and "Lectures on Biochemical Experiments, Second Series, 1, Methods in Gene Study III" edited by The Japanese Biochemical Society, published from Tokyo Kagaku Dojin, 1987).

Immunization may be accomplished by administration of the immunogen (the antigenic outermembrane protein as described above) via subcutaneous injection, intramuscular injection, oral administration, or the like. The amount of immunogen inoculated is preferably in the range of 0.01 to 10 mg on protein or peptide basis. A higher antibody titer is obtained by giving booster immunization at an interval of 2 to 10 weeks after the initial immunization. After 2 weeks from the booster, the antibody that specifically reacts with the avian coccidium antigen will be produced not only in the serum of the chicken but also in the egg. The thus immunized antibody will generally retain its high antibody titer for 4 months. If the antibody titer should decline, booster immunization may be given in a manner similar to the one as described above so that the titer will maintain its high level. Antibody activity of the egg can be evaluated by fluorescent antibody technique or enzyme antibody technique.

The immunogen is preferably injected as an admixture with an adjuvant. The adjuvant used may be the one known in the art such as Freund's complete adjuvant, Freund's incomplete adjuvant, aluminum hydroxide adjuvant, and Bordetella pertussis adjuvant.

The antibody in the thus produced egg can be used in the form of a solution of the whole egg or the egg yolk, or in the form of a powder prepared using a spray dryer or the like. The whole egg or the egg yolk may also be degreased for use in the form of a water-soluble protein. The antibody may also be purified or crudely purified before its use.

The composition of the present invention is administered to chickens. A dosage of the composition is generally in the range of 1 to 10⁶ folds in terms of antibody titer per day per one individual, although the amount administered is preferably adjusted depending on age by week, sex, breed, and the like of the bird.

When the antibody as described above is administered together with a lactic acid bacterium, the symptom of the body weight loss associated with the chicken coccidiosis is better relieved. The lactic acid bacterium may be generally used at a ratio of 10-10⁹ lactic acid bacteria to 1 g of the antibody. The lactic acid bacterium may be administered either simultaneously with the antibody, or at an appropriate interval from the administration of the antibody.

The lactic acid bacterium used for such purpose is not limited to any particular species, and any lactic acid bacterium belonging to Lactobacillus, Bifidobacterium, Streptococcus, Enterococcus, or Lactococcus may be used either alone or as a mixture of two or more species or strains. Examples of lactic acid bacteria belonging to the genus Lactobacillus include Lactobacillus acidophilus, L. bulgaricus, L. casei, L. fernentum, L. rhamnosus, L. paracasei, L. lactis, L. plantarum, L. reuteri, L. rhamnosus, and L. salivarius; the lactic acid bacteria belonging to genus Bifidobacterium include Bifidobacterium adolescentis, B. bifidum, B. breve, B. infantis, B. longum, B. pseudolongum, and B. thermophilum; the lactic acid bacteria belonging to genus Streptococcus include Streptococcus agalactiae, S. salivarius, and S. thermophilus; the lactic acid bacteria belonging to genus Enterococcus include Enterococcus faecalis and E. faecium; and the lactic acid bacteria belonging to the genus Lactococcus include Lactococcus lactis.

When the antibody as described above is administered together with an antibody from an egg of the chicken immunized with Clostridium perfringens (hereinafter referred to as "anti-Clostridium perfringens antibody"), the symptom of the body weight loss associated with the chicken coccidiosis is better relieved. The anti-Clostridium perfringens antibody may be generally used at a ratio of 0.01-100 g to 1 g of the anti-chicken coccidiosis antibody. The anti-Clostridium perfringens antibody may be administered either at the same time as with the anti-chicken coccidiosis antibody, or at an appropriate interval from the administration of the anti-chicken coccidiosis antibody.

The anti-Clostridium perfringens antibody may be prepared by a process similar to the process used for preparing the anti-chicken coccidiosis antibody as described above, except that the immunogen used is the Clostridium perfringens. The anti-Clostridium perfringens antibody and the anti-chicken coccidiosis antibody can also be produced at the same time by simultaneous immunization with the Clostridium perfringens and the antigenic outermembrane protein as described above.

Examples of antigens which may be used in producing the anti-Clostridium perfringens antibody include the protein described in J. Anim. Sci., 75: 19-25, 1997; the toxin described in Avian Diseases, 21: 241-255, 1977; the protein described in Vet. Rec., 120: 435-439, 1987; the protein described in Vaccine, 11: 1253-1258, 1993; and the protein described in FEMS Immunol. Med. Microbiol., 7: 321-336, 1993.

The anti-chicken coccidiosis composition of the present invention may be fed as a solution containing each effective component at a concentration of 0.001%-10%, or may be fed as powder, granules, tablets or paste which is mixed with a feed so that the each effective component is contained in the feed at a concentration of 0.001%-10%. Also, the anti-chicken coccidiosis antibody may be administered in the enteric coated dosage form to prevent digestion and decomposition of the composition in the stomach.

This specification includes the contents as disclosed in the specification of Japanese Patent Application No. 2002-189137, which is a priority document of the present application.

### Best Mode for Carrying Out the Invention

### [Example 1]

A 4 week old chicken was infected by orally administering 2 x 10⁶ oocysts of each of Eimeria acervulina NA strain (PE0101), Eimeria tenella NM strain (PE0102), and Eimeria maxima NT strain (PE0103) which are associated with chicken coccidiosis, and autopsy of the chicken was conducted at 4 days after the infection to obtain intestinal tract and its content. Purification of the sporozoit and the merozoite was conducted in accordance with Avian Diseases, 39: 538-547, 1995. Soluble outermembrane protein from the merozoite of Eimeria acervulina (NA strain) was subjected to SDS-PAGE to obtain outermembrane protein of 18 to 27 kD (Avian Diseases, 44: 379-389, 2000). A solution containing 0.5 mg/ml of this outermembrane protein was emulsified with Freund' s incomplete adjuvant, and the resulting emulsion was injected to 12 week old hen into its left and right pectoralis muscles at a dose of 1 ml for each muscle for initial immunization. 6 weeks after the initial immunization, the hen was given the second immunization in a similar manner. 2 weeks after the second immunization, antibody titer of the anti-chicken coccidiosis antibody in the blood of the hen was measured by enzyme-linked immunosorbent assay (ELISA) (Avian Diseases, 44: 379-389, 2000), and the antibody titer was 30, 000 to 120, 000 folds. The egg of this hen had an antibody titer of 30,000 to 120,000 folds, and this antibody titer level was maintained for another 4 months. The eggs were gathered, and a whole egg powder comprising an egg antibody was produced by spray drying. The powder produced had an antibody titer of 60, 000 folds. When this antibody was evaluated for its cross reactivity with the sporozoit and the merozoite by indirect immunofluorescent staining (Avian Diseases, 44: 379-389, 2000), this antibody specifically stained the surface of the sporozoit and the merozoite of the Eimeria species (Eimeria acervulina, Eimeria tenella, and Eimeria maxima) confirming that this antibody was a common antibody for these species.

### [Example 2]

The anti-chicken coccidiosis antibody produced in Example 1 is added to a broiler feed to evaluate effects of the antibody on the chickens that had been experimentally infected with chicken coccidiosis (Eimeria tenella) (Avian Disease, 31: 112-119, 1987). The animal used in this evaluation was "Chunky" which is a strain specifically developed for broiler production, and the antibody was administered by adding the antibody to the standard broiler feed for early fattening period (SDB No.1: Research Institute For Animal Science In Biochemistry & Toxicology) at a concentration of 0.1%, 1%, and 10%. The chickens were infected with oocysts of Eimeria tenella (ET strain) at 1000 oocysts/animal (J. Protozool, 9: 154-161, 1962), and each group includes 10 chickens. The observation period of the chickens after the infection was 2 weeks. The chickens were mainly observed for their weight gain and feed conversion ratio. O.P.G. (oocysts per gram of faeces) and cecal lesion were also observed ("Coccidiosis in Chicken", editorial supervision by K. Tsunoda, published by Chikusan Shuppan, 1983). Significant effects in the weight gain were then confirmed in the group that had been given the feed comprising 0.1% of the antigen, and significant effects in the feed conversion ratio were confirmed in the group that had been given the feed comprising 1.0% of the antigen.

As evidence from the results, by administration of the feed comprising 0.1% or more of the antibody to the chickens attacked by the chicken coccidiosis, the conditions of the chickens including the weight gain and the feed conversion ratio are significantly improved. The overall effect of the test substance was determined by using the scores of each observation, and the antibody proved to be very effective.

**Table 1**

| | Uninfected group | Group infected with *E. tenella* | | | |
|---|---|---|---|---|---|
| | Group with no addition to the feed | Proportion of the antibody added to the feed | | | |
| | 0% | 0% | 0.1% | 1% | 10% |
| Average weight gain (g) | 731 | 578 | 622 | 620 | 666 |
| Relative weight gain (%) ^{a)} | 100 | 79 | 85 | 85 | 91 |
| Average feed intake (g) | 879 | 885 | 910 | 862 | 915 |
| Feed conversion rate^{b)} | 1.20 | 1.53 | 1.46 | 1.39 | 1.37 |
| Suvival rate(%) | 100 | 100 | 100 | 100 | 100 |
| OPG (x 10⁴) at 7th day | 0.0 | 9.7 | 5.1 | 9.2 | 0.4 |
| Oocyst score^{c)} | 0 | 40 | 20 | 10 | 5 |
| Cecal lesion score^{d)} | 0 | 20 | 10 | 5 | 5 |
| ACI index^{e)} | 200 | 119 | 155 | 170 | 181 |
| | | | Slightly effective | Considerably effective | Very effective |

| | | | | | |
|---|---|---|---|---|---|
| a) : Relative weight gain (%) = [weight gain of the infected group] [weight gain of the uninfected group] x 100 b) : Feed conversion ratio = [average feed intake] ÷ [average weight gain] c) : Oocyst score of each group was determined by the calculation and the criteria as described below: O.P.G. of the medicated group in relation to the control group = [O.P.G. of the medicated group] ÷ [O.P.G. of the infected unmedicated control group) x 100 Criteria: 0: 0 to 1%; +5: 1.1 to 25%; +10: 26 to 50%; +20: 51 to 75%; +40: 76 to 100%. d) : Cecal lesion score was determined by the following criteria: 0: no lesion observed; +1: a very small number of ecchymosis are scattered along the cecal wall, while the content is normal; +2: small amount of blood is found in the content, and a large number of bleeding lesions are observed; +3: massive hemorrhage or cecal core (cheesy mass of banana-like shape either with blood clots or pale gray color) is found in the cecum, and thickening of cecal wall as well as deformation and atrophy of the cecum are clearly observed; +4: cecum undergoes marked atrophy, and the lesion extends to the rectum. The wall is thickened to the extremity, and blood clotting or cecal core is found in the cecum. Average score was determined for 1 chicken, and the score converted to that of 10 chickens are shown in Table 1. e): ACI (Anti-coccidial Index) = [relative percent weight gain + suvival rate] - [oocyst score + lesion score] | | | | | |

ACI: 180 or higher: very effective; 160 to 179: considerably effective; 120 to 161: slightly effective; less than 120: not effective.

### [Example 3]

Effect of the antibody on the chicken which had been experimentally infected with chicken coccidiosis (Eimeria tenella) was evaluated by using a feed comprising the anti-chicken coccidiosis antibody produced in Example 1 and a lactic acid bacterium (Lactobacillus acidophilus). The lactic acid bacterium (Lactobacillus acidophilus, ATCC 4356 strain) was cultivated in MRS medium, and a powder including 1 x 10¹¹ bacteria/g was produced by lyophilization. The animal used in this evaluation was "Chunky" which is a broiler strain, and the chickens were fed with a standard broiler feed for early fattening period (SDB No.1) having the 0.1% of the antibody and 10⁶/g of lactic acid bacterium added thereto. The chickens were infected with oocysts of Eimeria tenella (ET strain) at 1000 oocysts/animal, and each group includes 10 chickens. The observation period of the chickens after the infection was 2 weeks. The chickens were mainly observed for their weight gain and feed conversion ratio. O.P.G. and cecal lesion were also observed. Significant effects were confirmed in the weight gain in the group that had been administered with the antibody and the lactic acid bacterium. Significant effects were also confirmed in the feed conversion ratio in the same group.

As evidence from the results, by administration of the feed comprising the anti-chicken coccidiosis antibody and the lactic acid bacterium (Lactobacillus acidophilus) to the chickens attacked by the chicken coccidiosis, the conditions of the chickens including the weight gain and the feed conversion ratio are significantly improved. The overall effect of the test substance was determined by using the scores of each observation, and the antibody proved to be very effective.

**Table 2**

| | Uninfected group | Group infected with *E. tenella* | | |
|---|---|---|---|---|
| | Group with no addition to the feed | Addition to the feed | | |
| | Control group | Control group | 0.1% of antibody | antibody + lactic acid bacterium |
| Average weight gain (g) | 731 | 578 | 622 | 666 |
| Relative weight gain (%) | 100 | 79 | 85 | 91 |
| Average feed intake (g) | 879 | 885 | 910 | 845 |
| Feed conversion rate | 1.20 | 1.53 | 1.46 | 1.27 |
| Suvival rate(%) | 100 | 100 | 100 | 100 |
| OPG (x 10⁴) at 7th day | 0.0 | 9.7 | 5.1 | 2.2 |
| Oocyst score | 0 | 40 | 20 | 5 |
| Cecal lesion score | 0 | 20 | 10 | 5 |
| ACI index | 200 | 119 | 155 | 181 |
| | | | Slightly effective | Very effective |

### [Example 4]

Effect of the feed comprising the anti-chicken coccidiosis antibody produced in Example 1 and the anti-Clostridium perfringens antibody produced from the egg of a chicken immunized with Clostridium perfringens was evaluated on the chicken which had been experimentally infected with chicken coccidiosis (Eimeria tenella) and Clostridium perfringens (Avian Disease, 24: 324-333, 1980).

Anti-Clostridium perfringens antibody was produced as described below. Clostridium perfringens (ATCC 3624 strain) was cultivated in thioglycolate medium. A solution containing 1 x 10¹⁰/ml of the Clostridium perfringens was emulsified with Freund's incomplete adjuvant, and the resulting emulsion was injected to 12 week old hen into its left and right pectoralis muscles at a dose of 1 ml for each muscle for initial immunization. 6 weeks after the initial immunization, the hen was given the second immunization in a similar manner. 2 weeks after the second immunization, antibody titer of the anti-Clostridium perfringens antibody in the blood of the hen was measured by cell agglutination test, and the antibody titer was 300 to 1200 folds. The egg of this hen had an antibody titer of 300 to 1200 folds, and this antibody titer level was maintained for another 4 months. The eggs were gathered, and a whole egg powder comprising an egg antibody was produced by spray drying. The powder produced had an antibody titer of 600 folds.

The animal used in this evaluation was "Chunky" which is a broiler strain, and the chickens were fed with a standard broiler feed for early fattening period (SDB No.1) having the 0.1% of the anti-chicken coccidiosis antibody and 0.1% of anti-Clostridium perfringens antibody added thereto. The chickens were infected with oocysts of Eimeria tenella (ET strain) at 1000 oocysts/animal, and infected with clostridium perfringens (ATCC 3624 strain) at 10⁷ of the Clostridium perfringens/animal, and each group includes 10 chickens. The observation period of the chickens after the infection was 2 weeks. The chickens were mainly observed for their weight gain and feed conversion ratio. O.P.G. and cecal lesion were also observed. Significant effects were confirmed in the weight gain in the group that had been administered with both the anti-chicken coccidiosis antibody and the anti-Clostridium perfringens antibody. Significant effects were also confirmed in the feed conversion ratio in the same group.

As evidence from the results, by simultaneous administration of 0.1% of the anti-chicken coccidiosis antibody and 0.1% of the anti-Clostridium perfringens antibody in a feed to the chickens attacked by both the chicken coccidiosis and the Clostridium perfringens, the conditions of the chickens including the weight gain and the feed conversion ratio are significantly improved. The overall effect of the test substance was determined by using the scores of each observation, and these antibodies proved to be very effective.

**Table 3**

| | Uninfected group | Group infected with *E. tenella* and Clostridium | | |
|---|---|---|---|---|
| | Group with no addition to the feed | Addition to the feed | | |
| | Control group | Control group | 0.1% of anti chicken coccidiosis antibody | 0.1% of chicken coccidiosis antibody + 0.1% of anti Clostridium antibody |
| Average weight gain (g) | 731 | 563 | 620 | 629 |
| Relative weight gain | 100 | 77 | 85 | 86 |
| Average feed intake (g) | 879 | 880 | 905 | 865 |
| Feed conversion rate | 1.20 | 1.56 | 1.46 | 1.38 |
| Suvival rate(%) | 100 | 100 | 100 | 100 |
| OPG (x 10⁴) at 7th day | 0.0 | 12.0 | 6.3 | 0.1 |
| Oocyst score | 0 | 40 | 20 | 0 |
| Cecal lesion score | 0 | 24 | 12 | 3 |
| ACI index | 200 | 113 | 153 | 183 |
| | | | Slightly effective | Very effective |

### [Example 5]

The anti-chicken coccidiosis antibody produced in Example 1 is added to a feed to evaluate effects of the antibody on the chickens that had been experimentally infected with chicken coccidiosis (Eimeria acervulina) (Avian Disease, 31: 112-119, 1987). The animal used in this evaluation was "Chunky" which is a broiler strain, and the antibody was administered by adding the antibody to the standard broiler feed for early fattening period (SDB No.1) at a concentration of 0.1%, 1%, and 10%. The chickens were infected with oocysts of Eimeria acervulina (EA strain) at 1000 oocysts/animal, and each group includes 10 chickens. The observation period of the chickens after the infection was 2 weeks. The chickens were mainly observed for their weight gain and feed conversion ratio. O.P.G. and small intestine lesion were also observed. Significant effects of the antibody administration was confirmed in the weight gain even in the group that had been given the feed comprising 0.1% of the antibody.

As evidence from the results, by administration of the feed comprising the anti-chicken coccidiosis antibody at a rate of 0.1% or more to the chickens attacked by the chicken coccidiosis, the weight gain of the chickens is significantly improved.

**Table 4**

| | Uninfected group | Group infected with *E. acervulina* | | | |
|---|---|---|---|---|---|
| | Group with no addition to the feed | Proportion of the antibody added to the feed | | | |
| | 0% | 0% | 0.1% | 1% | 10% |
| Average weight gain (g) | 731 | 580 | 630 | 650 | 680 |
| Relative weight gain (%) | 100 | 79 | 86 | 89 | 93 |
| Average feed intake (g) | 879 | 826 | 869 | 845 | 820 |
| Feed conversion rate | 1.20 | 1.4 | 1.38 | 1.30 | 1.22 |
| Suvival rate(%) | 100 | 100 | 100 | 100 | 100 |
| OPG (x 10⁴) at 7th day | 0.0 | 5.0 | 4.5 | 4.0 | 0.3 |
| Oocyst score | 0 | 40 | 40 | 40 | 5 |
| Small intestine lesion score^{a)} | 0 | 20 | 5 | 5 | 0 |
| ACI index^{e)} | 200 | 119 | 141 | 144 | 188 |
| | | | Slightly effective | Slightly effective | Very effective |

| | | | | | |
|---|---|---|---|---|---|
| a): Small intestine lesion score was determined by the following criteria: | | | | | |

0: no lesion observed; +1: white necrotic lesions containing oocysts are scattered in duodenum; +2: an increased number of white necrotic lesions are observed, and the lesion extends to the upper small intestine; +3: an even more increased number of lesions are found, and the lesions are fused with each other. Thickening of the small intestine wall is observed, and the content is watery; +4: duodenum and upper small intestine have become grayish because of the large number of mutually fused lesions. The wall is extremely thickened, and creamy mucus is contained in the interior of the wall. Average score was determined for 1 chicken, and the score converted to that of 10 chickens are shown in Table 4.

As shown in Examples 2, 3, and 5, when the anti-chicken coccidiosis antibody or the antibody combined with the lactic acid bacterium was continuously administered to the chicken by mixing them in a feed, symptoms of the chicken experimentally infected with chicken coccidiosis were ameliorated, and the chicken showed an increased weight gain. When the chicken infected with chicken coccidiosis was administered with a mixture of the anti-chicken coccidiosis antibody and the lactic acid bacterium by mixing them in a feed, the conditions of the chicken were remarkably improved including the improvement in the weight gain and the feed conversion ratio. When the anti-Clostridium perfringens antibody was simultaneously administered with the anti-chicken coccidiosis antibody, development of the chicken coccidiosis was prevented, and improvements in the conditions including weight gain and the feed conversion ratio were recognized.

### Industrial Applicability

The anti-chicken coccidiosis composition of the present invention comprising an anti-chicken coccidiosis antibody optionally with a lactic acid bacterium or an anti-Clostridium perfringens antibody specifically reacts with coccidium in chicken. Therefore, the anti-chicken coccidiosis composition of the present invention is very effective in preventing coccidiosis in chicken, and can be used as a substitute for the anticoccidial drugs and vaccines. The composition is also quite effective in ameliorating the symptoms of chicken coccidiosis and in the weight gain.

## Claims

1. A composition for oral administration, which is used for prevention or treatment of chicken coccidiosis, comprising an antibody that is directed against a soluble outer membrane protein of 18 to 27 kD from the merozoite of Eimeria acervulina and obtained from an egg of a chicken immunized with the soluble outer membrane protein of 18 to 27 kD from the merozoite of Eimeria acervulina.

2. A composition according to claim 1 further comprising a lactic acid bacterium.

3. A composition according to claim 1 or 2 further comprising an anti-Clostridium perfringens antibody obtained from an egg of a chicken immunized with Clostridium perfringens.

4. An avian feed comprising the composition according to any one of claims 1 to 3.

5. Use of an antibody that is directed against a soluble outer membrane protein of 18 to 27 kD from the merozoite of Eimeria acervulina and obtained from an egg of a chicken immunized with a soluble outer membrane protein of 18 to 27 kD from the merozoite of Eimeria acervulina in manufacturing a pharmaceutical composition for oral administration or an avian feed for preventing or treating chicken coccidiosis.

## Patentansprüche

1. Zusammensetzung zur oralen Verabreichung, die zur Prävention oder Behandlung von Hühner-Kokzidiose verwendet wird, umfassend einen Antikörper, der gegen ein lösliches Protein der äußeren Membran mit 18 bis 27 kD aus dem Merozoiten von *Eimeria acervulina* gerichtet ist und erhalten wird aus dem Ei eines Huhns, das mit dem löslichen Protein der äußeren Membran mit 18 bis 27 kD aus dem Merozoiten von *Eimeria acervulina* immunisiert ist.

2. Zusammensetzung nach Anspruch 1, des Weiteren umfassend ein Milchsäure-Bakterium.

3. Zusammensetzung nach Anspruch 1 oder 2, des Weiteren umfassend einen Antikörper gegen *Clostridium perfringens*, der erhalten wird aus dem Ei eines mit *Clostridium perfringens* immunisierten Huhns.

4. Vogelfutter, umfassend die Zusammensetzung nach einem der Ansprüche 1 bis 3.

5. Verwendung eines Antikörpers, der gegen ein lösliches Protein der äußeren Membran mit 18 bis 27 kD aus dem Merozoiten von *Eimeria acervulina* gerichtet ist und erhalten wird aus dem Ei eines Huhns, das mit dem löslichen Protein der äußeren Membran mit 18 bis 27 kD aus dem Merozoiten von *Eimeria acervulina* immunisiert ist, zur Herstellung einer pharmazeutischen Zusammensetzung zur oralen Verabreichung oder eines Vogelfutters zur Prävention oder Behandlung von Hühner-Kokzidiose.

## Revendications

1. Composition pour administration par voie orale qui est utilisée pour la prévention ou le traitement de la coccidiose du poulet, comprenant un anticorps qui est dirigé contre une protéine membranaire extérieure soluble de 18 à 27 kD du mérozoïte d'Eimeria acervulina et obtenu à partir d'un oeuf de poule immunisée avec la protéine membranaire extérieure soluble de 18 à 27 kD du mérozoïte d'Eimeria acervulina.

2. Composition selon la revendication, 1 comprenant en plus une bactérie d'acide lactique.

3. Composition selon la revendication 1 ou 2, comprenant en plus un anticorps anti-Clostridium perfringens obtenu à partir d'un oeuf d'une poule immunisée avec du Clostridium perfringens.

4. Nourriture aviaire comprenant la composition selon l'une des revendications 1 à 3.

5. Utilisation d'un anticorps qui est dirigé contre une protéine membranaire extérieure soluble de 18 à 27 kD du mérozoïte d'Eimeria acervulina et obtenu à partir d'un oeuf de poule immunisée avec la protéine membranaire extérieure soluble de 18 à 27 kD du mérozoïte d'Eimeria acervulina pour préparer une composition pharmaceutique pour administration par voie orale ou une nourriture aviaire, destinée à la prévention ou au traitement de la coccidiose du poulet.
